# EUROPEAN PATENT APPLICATION

(11) **EP 4 813 324 A1**
(43) Date of publication of application: **30.09.2026**
(21) Application number: 25858874.8
(22) Date of filing: 01.08.2025
(51) Int. Cl.: A61B 17/34, A61B 8/08, A61B 34/20, A61B 34/30, A61B 34/00

(54) **CALIBRATION METHOD, CALIBRATION DEVICE, NON-VOLATILE STORAGE MEDIUM, AND SURGICAL OPERATION DEVICE**

(30) Priority: 28.08.2024 CN 202411194143
(71) Applicant: Beijing Easy Surg Medical Technology Co., Ltd., Beijing 100085 (CN)
(72) Inventor: FU, Jianguang, Beijing 100085 (CN); LIU, Wenyong, Beijing 100085 (CN); YAO, Lin, Beijing 100085 (CN); MI, Kuan, Beijing 100085 (CN)
(74) Representative: Bandpay & Greuter
(86) International application number: PCT/CN2025/112074
(87) International publication number: WO 2026/045830

(57) **Abstract**

The present invention provides a calibration method, a calibration device, a non-volatile storage medium, and a surgical operation device, wherein the calibration method comprises: obtaining a first transformation matrix $T P F$ between a base coordinate system {F} of the surgical operation device and a coordinate system {P} of a calibration module mounted on a base of the surgical operation device; performing ultrasound imaging of the calibration module using an ultrasound probe, and extracting features from an ultrasound image; mapping the features of the ultrasound image to corresponding actual three-dimensional features to obtain a second transformation matrix $T l F$ between a coordinate system {I} of the ultrasound image and the base coordinate system {F} of the surgical operation device, and determining, based on the second transformation matrix $T l F$*,* a positional deviation of the base of the surgical operation device relative to the physical space of the ultrasound probe. The technical solution provided by the present invention solves the technical problem in the prior art that deviations in clamping angle and position of an ultrasound probe significantly affect puncture accuracy.

## Description

### Field of the Invention

The present invention relates to the technical field of surgical operation device, in particular to a calibration method, a calibration device, a non-volatile storage medium, and a surgical operation device.

### Background to the Invention

Currently, percutaneous puncture is a minimally invasive surgical procedure widely used in tumor-related applications such as biopsy, ablation, and seed implantation. Assisted by medical imaging, a puncture needle is inserted percutaneously into a target region to establish a feasible access pathway for interventional procedures or biopsy sampling, thereby enabling diagnosis or treatment of diseases. In a conventional puncture procedure, under ultrasound guidance, a physician typically holds an ultrasound probe in one hand and a puncture needle in the other hand, identifies a lesion region based on ultrasound images, and manually advances the puncture needle into a patient's body.

However, in the prior art, due to differences in the shapes of B-mode ultrasound probes used in different hospitals, angular and positional deviations of the B-mode ultrasound probe may occur during clamping of the B-mode ultrasound probe. Since a surgical robot uses B-mode ultrasound images as a positioning reference, deviations in the clamping angle and position of the B-mode ultrasound probe may significantly affect puncture accuracy.

### Statement of Invention

A primary objective of the present invention is to provide a calibration method, a calibration device, a non-volatile storage medium, and a surgical operation device, so as to solve the technical problem in the prior art that deviations in the clamping angle and position of an ultrasound probe significantly affect puncture accuracy.

To achieve the above objective, according to a first aspect of the present invention, a calibration method for an ultrasound probe of a surgical operation device is provided, comprising:
obtaining a first transformation matrix $T P F$ between a base coordinate system {F} of the surgical operation device and a coordinate system {P} of a calibration module mounted on a base of the surgical operation device;
performing ultrasound imaging of the calibration module using an ultrasound probe, and extracting features from an ultrasound image;
mapping the features of the ultrasound image to corresponding actual three-dimensional features to obtain a second transformation matrix $T l F$ between a coordinate system {I} of the ultrasound image and the base coordinate system {F} of the surgical operation device, and determining, based on the second transformation matrix $T l F$, a positional deviation of the base of the surgical operation device relative to the physical space of the ultrasound probe.

Further, obtaining the first transformation matrix $T P F$ between the base coordinate system {F} of the surgical operation device and the coordinate system {P} of the calibration module mounted on the base of the surgical operation device comprises:
obtaining the base coordinate system {F} of the surgical operation device and a Marker coordinate system {M} of a visual Marker mounted on the surgical operation device, so as to obtain a third transformation matrix $T M F$ between the Marker coordinate system and the base coordinate system of the surgical operation device;
obtaining the coordinate system {P} of the calibration module mounted on the base of the surgical operation device, so as to obtain a fourth transformation matrix $T P M$ between the coordinate system {P} of the calibration module and the Marker coordinate system {M};
obtaining, based on the third transformation matrix $T M F$ and the fourth transformation matrix $T P M$, the first transformation matrix $T P F$ between the coordinate system {P} of the calibration module and the base coordinate system {F} of the surgical operation device.

Further, mapping the features of the ultrasound image to corresponding actual three-dimensional features to obtain a second transformation matrix $T l F$ between the coordinate system {I} of the ultrasound image and the base coordinate system {F} of the surgical operation device, and determining, based on the second transformation matrix $T I F$, positional deviations of the base of the surgical operation device relative to the ultrasound image space and the physical space, comprises:
setting pixel scaling factors of the ultrasound image as Sx and Sy;
setting **(*u*,*v*)** as pixel coordinates in the ultrasound image coordinate system {I}, and setting **(*x*, *y*, *z*)** as three-dimensional coordinates corresponding to **(*u*,*v*)** in the coordinate system {P} of the calibration module:
   establishing the following formula: $\left[\begin{matrix}x \\ y \\ z \\ 1\end{matrix}\right] = T - 1 P F T l F \left[\begin{matrix}{s}_{x} u \\ {s}_{y} v \\ 0 \\ 1\end{matrix}\right] .$

Further, the calibration module is an N-wire module; mapping the features of the ultrasound image to corresponding actual three-dimensional features to obtain a second transformation matrix $T I F$ between the coordinate system {I} of the ultrasound image and the base coordinate system {F} of the surgical operation device, and determining, based on the second transformation matrix $T I F$, positional deviations of the base of the surgical operation device relative to the ultrasound image space and the physical space, further comprises:
designating three intersection points between the ultrasound imaging plane and the three line segments of each N-shaped structure of the N-wire module as point P1, point P2, and point P3, respectively, and obtaining coordinates ***P*_{*A*1}, *P*_{A2},*P*_{*B*1}, *P*_{*B*2}** of four endpoints A1, A2, B1, and B2 of each N-shaped structure in the coordinate system {P} of the calibration module; wherein the point P1, the point P2, and the point P3 are respectively intersection points between the ultrasound imaging plane and a line on a first side of the N-shape, a middle line of the N-shape, and a line on a second side of the N-shape;
extracting pixel coordinates ***p*₁,*p*₂,*p*₃** of the point P1, the point P2, and the point P3 in the coordinate system {I} of the ultrasound image, respectively;
calculating coordinates ***P*₂** of the point P2 in the coordinate system {P} of the calibration module according to a similar-triangle principle,
wherein ${P}_{2} = \left(1-λ\right) {P}_{B 1} + λ {P}_{A 2} ; λ = \frac{\left‖{p}_{1}-{p}_{2}\right‖}{\left‖{p}_{1}-{p}_{3}\right‖} ;$
wherein two-dimensional pixel coordinates and three-dimensional coordinates of the point P2 in the model coordinate system {P} are respectively represented by ***pᵢ*** = **[*uᵢ*,*vᵢ*]^{T}** and ***Pᵢ*, *i*** = **1, ···,*K*;** wherein K denotes a quantity of N-wires;
setting R and t respectively as a rotation matrix and a translation vector of the first transformation matrix $T P F$, such that the following relationship is derived: ${s}_{x} {u}_{i} {r}_{1} + {s}_{y} {v}_{i} {r}_{2} + t = {y}_{i} ;$
wherein, ***r***₁ and ***r***₂ are first two columns of a rotation component ***R*** of the second transformation matrix $T I F$, and ***t*** is a translation vector of $T I F$, ***y**ᵢ = **R P**ᵢ **+ t***; let ***a***₁ = *sₓ**r***₁,***a***₂ = *s_{y}**r***₂, $x = {\left[{a}_{1}^{T}, {a}_{2}^{T}, {t}^{T}\right]}^{T}$, and the above formula is rewritten in matrix form as: ${A}_{i} x = {y}_{i}$
where: ${A}_{i} = \left[\begin{matrix}{u}_{i} & 0 & 0 & {ν}_{i} & 0 & 0 & 1 & 0 & 0 \\ 0 & {u}_{i} & 0 & 0 & {ν}_{i} & 0 & 0 & 1 & 0 \\ 0 & 0 & {u}_{i} & 0 & 0 & {ν}_{i} & 0 & 0 & 1\end{matrix}\right] ;$
stacking all ***Aᵢ*** and ***yᵢ*** (*i* **= 1, ···,*K*)** row-wise to form a final linear equation ***Ax*** = ***y***, a least-squares solution thereof being ***x*** = **(*A^{T}A*)⁻¹*A^{T}y*;**
after ***x*** is determined, ***a***₁***, a***₂, and ***t*** are obtained accordingly;
after calculation, ***sₓ*** = ||***a*₁**||, ***s_{y}*** = ||***a*₂**||, ***r*₁ = *a*₁/*sₓ***, ***r*₂ = *a*₂/*s*_{y}**, and ***r*₃ = *r*₁ × *r*₂** are obtained so as to calculate the second transformation matrix.

Further, after obtaining ***sₓ*** = ||***a*₁**||, ***s_{y}*** = ||***a*₂**||, ***r*₁ = *a*₁/*sₓ***, ***r*₂ = *a*₂/*s_{y}***, and ***r*₃ = *r*₁ × *r*₂** through calculation so as to calculate the second transformation matrix, the calibration method further comprises:
calculating a root-mean-square error $RMSE = \sqrt{\frac{1}{n} {\sum }_{i = 1}^{n} {\left‖{{T}_{FI}}^{I}{P}_{i}-P i F\right‖}^{2}}$;
defining outliers according to {∪***P**ᵢ*| ∥***T**_{FP}* ^{P}***P**ᵢ* - *^{F}**P**ᵢ*∥ > *RMSE*}, and removing outliers satisfying the outlier definition formula from original data;
recalculating *sₓ* and *s_{y},* and recalculating to obtain ***T**_{FP}.*

According to a second aspect of the present invention, a calibration device for a surgical operation device is provided, comprising:
an acquisition module configured to obtain a first transformation matrix $T P F$ between a base coordinate system {F} of the surgical operation device and a coordinate system {P} of a calibration module mounted on a base of the surgical operation device;
an ultrasound imaging module configured to perform ultrasound imaging of the calibration module using an ultrasound probe and to extract features from an ultrasound image; and
a computation module, configured to map the features of the ultrasound image to corresponding actual three-dimensional features to obtain a second transformation matrix $T I F$ between an ultrasound image coordinate system {I} and the base coordinate system {F} of the surgical operation device, and determine, based on the second transformation matrix $T I F$, a positional deviation of the base of the surgical operation device relative to the physical space of the ultrasound probe.

Further, the computation module comprises:
a first setting module, configured to set pixel scaling factors of the ultrasound image as Sx and Sy;
a second setting module, configured to set **(*u*,*v*)** as pixel coordinates in the coordinate system {I} of the ultrasound image, and to set **(*x*, *y, z*)** as three-dimensional coordinates corresponding to **(*u*,*v*)** in the coordinate system {P} of the calibration module:
   a formula establishment module, configured to establish the following formula: $\left[\begin{matrix}x \\ y \\ z \\ 1\end{matrix}\right] = T - 1 P F T I F \left[\begin{matrix}{s}_{x} u \\ {s}_{y} v \\ 0 \\ 1\end{matrix}\right] .$

According to a third aspect of the present invention, a non-volatile storage medium is provided, wherein the non-volatile storage medium stores a plurality of instructions, the instructions being configured to be loaded and executed by a processor so as to cause the processor to perform the calibration method described above.

According to a fourth aspect of the present invention, a surgical operation device configured for use with the calibration method described above is provided, wherein the surgical operation device comprises:
an operation member movably disposed;
an ultrasound probe mounted on the operation member; and
a calibration module detachably mounted on the operation member, wherein the calibration module comprises a calibration wire having a plurality of N-shaped structures, the calibration wire being disposed opposite the ultrasound probe.

Further, the calibration module further comprises:
a mounting body detachably mounted on the operation member, wherein the mounting body comprises a first mounting sidewall and a second mounting sidewall oppositely disposed, the first mounting sidewall being provided with a plurality of first threading holes, and the second mounting sidewall being provided with a plurality of second threading holes, the calibration wire being threaded back and forth between the plurality of first threading holes and the plurality of second threading holes to form a plurality of N-shaped structures.

Further, the calibration module further comprises:
a first detachable arm and a second detachable arm spaced apart from each other, wherein the first detachable arm and the second detachable arm are oppositely arranged on two sides of the mounting body, respectively configured to be snap-fitted to two sides of the ultrasound probe and detachably connected thereto, and the mounting body is positioned below a detection head of the ultrasound probe.

Further, the ultrasound probe comprises a mounting member and a probe portion connected to each other, wherein the mounting member is provided with a mounting channel, the probe portion is mounted on the mounting member, a portion of the probe portion is within the mounting channel, and another portion of the probe portion extends out of the mounting channel; the mounting member comprises a fixed end and an openable end spaced apart from each other, the openable end being rotatably disposed relative to the fixed end;
wherein contact surfaces of the fixed end and the openable end coincide with a central plane of the probe portion; and/or
the surgical operation device further comprises a puncture needle, wherein the puncture needle is positioned on the central plane of the probe portion.

Further, the surgical operation device further comprises a puncture needle, wherein the operation member comprises:
an angle control module, wherein a drive portion of the angle control module is rotatably disposed relative to a body portion of the angle control module, and the ultrasound probe is mounted on the body portion of the angle control module;
a depth control module, wherein the drive portion of the angle control module is drivingly connected to the depth control module, a drive end of the depth control module is movably disposed along a preset direction, and the drive end of the depth control module is drivingly connected to the puncture needle.

Further, the angle control module comprises a slider and a connecting rod, wherein the slider is movably disposed on the body portion of the angle control module along a preset direction; the connecting rod forms the drive portion of the angle control module, wherein one end of the connecting rod is hingedly connected to the slider, and the other end thereof is hingedly connected to the depth control module; and/or
the depth control module comprises a lead screw guide rail module and a drive block, wherein the lead screw guide rail module is rotatably disposed, and the drive block is mounted on the lead screw guide rail module and is threadedly engaged therewith, such that rotation of the lead screw guide rail module drives the drive block to move along a preset direction.

Further, the surgical operation device further comprises a micro-adjustment control module mounted on the operation member, wherein the micro-adjustment control module is configured to adjust the angle control module and the depth control module; and/or
the operation member has an initial state and an operational state for puncture; when the operation member is in the initial state, the angle control module and the depth control module are arranged in abutment with each other.

Further, the surgical operation device further comprises a puncture needle and a quick-release structure, wherein the puncture needle is mounted on the quick-release structure, and the quick-release structure is detachably mounted on the operation member.

Further, the quick-release structure comprises a base and a quick-release plate, wherein the quick-release plate is movably disposed on the base so as to move between a clamping position, in which the quick-release plate is disposed opposite at least a portion of the base and clamps the puncture needle, and a release position, in which the quick-release plate is displaced away from the base;
wherein the base is provided with a first snap-fit structure, and the quick-release plate is provided with a second snap-fit structure adapted to the first snap-fit structure; when the quick-release plate is in the clamping position, the first snap-fit structure is snap-engaged with the second snap-fit structure; the quick-release structure further comprises an elastic member disposed on the first snap-fit structure or the second snap-fit structure, the elastic member being positioned between the first snap-fit structure and the second snap-fit structure; and/or
the puncture needle comprises a mounting portion and a needle body, wherein the base is provided with a first mounting groove adapted to the mounting portion, and the quick-release plate is provided with a second mounting groove adapted to the mounting portion; when the quick-release plate moves to the clamping position, the first mounting groove and the second mounting groove are joined together to form an enclosure around the periphery of the mounting portion, and the needle body extends out of the quick-release structure.

Further, the surgical operation device further comprises:
an isolation plate detachably mounted on the operation member, wherein the puncture needle is detachably mounted on a side of the isolation plate away from the operation member.

By applying the technical solution of the present invention, the obtained first transformation matrix $T P F$ facilitates calculation of the second transformation matrix $T I F$ based on the second transformation matrix, thereby enabling determination, based on the second transformation matrix, of a positional deviation of the base of the surgical operation device relative to the physical space of the ultrasound probe caused by deviations in the clamping angle and position, thereby improving puncture accuracy of the ultrasound probe and enhancing operational accuracy of the surgical operation device. Therefore, the technical solution provided by the present invention solves the technical problem in the prior art that deviations in clamping angle and position of an ultrasound probe significantly affect puncture accuracy.

### Brief Description of the Drawings

The accompanying drawings, which form a part of the present application, are provided for further understanding of the present invention. The illustrative embodiments of the present invention and the descriptions thereof are intended to explain the present invention and shall not be construed as improperly limiting the present invention. In the accompanying drawings:
FIG. 1 is a flow chart of a calibration method according to an embodiment of the present invention;
FIG. 2 is a schematic diagram showing positions of four coordinate systems of a surgical operation device according to an embodiment of the present invention;
FIG. 3 is a schematic structural diagram of a surgical operation device according to an embodiment of the present invention;
FIG. 4 is a schematic structural diagram of an ultrasound probe according to an embodiment of the present invention;
FIG. 5 is a schematic structural diagram of a calibration module according to an embodiment of the present invention;
FIG. 6 is a schematic diagram showing intersection points between an ultrasound imaging plane of the ultrasound probe and N-shaped wires according to an embodiment of the present invention;
FIG. 7 is a schematic diagram showing the intersection points between the ultrasound imaging plane of the ultrasound probe and the N-wires from another perspective according to an embodiment of the present invention;
FIG. 8 is an ultrasound image obtained by the ultrasound probe according to an embodiment of the present invention;
FIG. 9 is a schematic structural diagram of the surgical operation device in an initial position according to an embodiment of the present invention;
FIG. 10 is a schematic structural diagram of the surgical operation device when an angle control module thereof is rotated by a predetermined angle according to an embodiment of the present invention;
FIG. 11 is a schematic structural diagram of a depth control module according to an embodiment of the present invention;
FIG. 12 is a schematic structural diagram of a quick-release structure according to an embodiment of the present invention;
FIG. 13 is a schematic structural diagram of the quick-release structure in an opened state according to an embodiment of the present invention;
FIG. 14 is a schematic structural diagram of an isolation plate according to an embodiment of the present invention.

In the drawings, the following reference numerals are used:
10. operation member; 11. angle control module; 111. body portion; 112. slider; 113. connecting rod; 114. motor; 115. lead screw guide rail assembly; 12. depth control module; 121. lead screw guide rail module; 122. drive block; 123. driving motor; 124. driving timing pulley; 125. timing belt; 126. driven timing pulley;
20. ultrasound probe; 21. mounting member; 211. mounting channel; 22. probe portion;
30. calibration module; 31. calibration wire; 32. mounting body; 33. first detachable arm; 34. second detachable arm;
40. puncture needle; 41. mounting portion; 42. needle body;
50. micro-adjustment control module;
60. quick-release structure; 61. base; 611. first mounting groove; 62. quick-release plate; 621. second mounting groove; 63. first snap-fit structure; 64. second snap-fit structure; 65. elastic member;
70. isolation plate; 71. adapter plate; 72. plug-in structure; 73. pressing structure.

### Detailed Description of the Embodiments

It should be noted that, unless otherwise contradictory, the embodiments of the present application and features thereof may be combined with each other. The present invention will be described in detail below by reference to the accompanying drawings and embodiments.

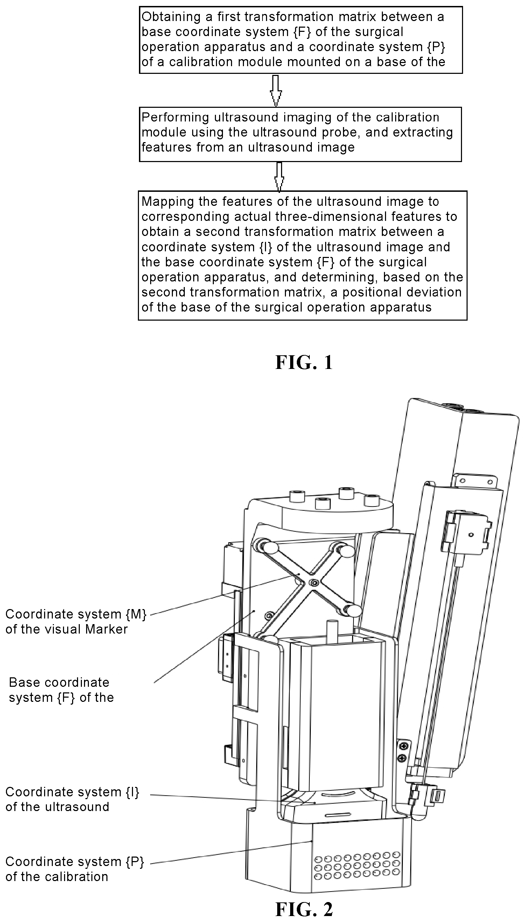

As shown in FIG. 1, Embodiment 1 of the present invention provides a calibration method for an ultrasound probe of a surgical operation device, comprising: obtaining a first transformation matrix $T P F$ between a base coordinate system {F} of the surgical operation device and a coordinate system {P} of a calibration module mounted on a base of the surgical operation device; performing ultrasound imaging of the calibration module using an ultrasound probe, and extracting features from an ultrasound image; mapping the features of the ultrasound image to corresponding actual three-dimensional features to obtain a second transformation matrix $T I F$ between a coordinate system {I} of the ultrasound image and the base coordinate system {F} of the surgical operation device, and determining, based on the second transformation matrix $T I F$, a positional deviation of the base of the surgical operation device relative to the physical space of the ultrasound probe.

By adopting the calibration method provided in the present embodiment, the obtained first transformation matrix $T P F$ facilitates calculation of the second transformation matrix $T I F$, thereby enabling determination, based on the second transformation matrix, of a positional deviation of the base of the surgical operation device relative to the physical space of the ultrasound probe caused by deviations in the clamping angle and position, thereby improving puncture accuracy of the ultrasound probe and enhancing operational accuracy of the surgical operation device.

In addition, since the calibration module is pre-calibrated with the first transformation matrix in advance during the above process, the second transformation matrix can be rapidly obtained after installation and calculation of the calibration module. Accordingly, deviations in physical space corresponding to the clamping angle and position of the ultrasound probe can be determined according to the second transformation matrix, thereby effectively improving operational accuracy of the surgical operation device.

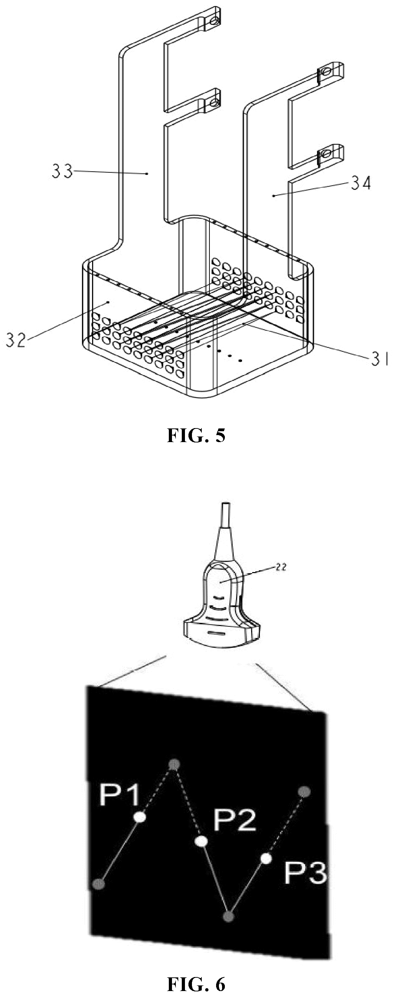

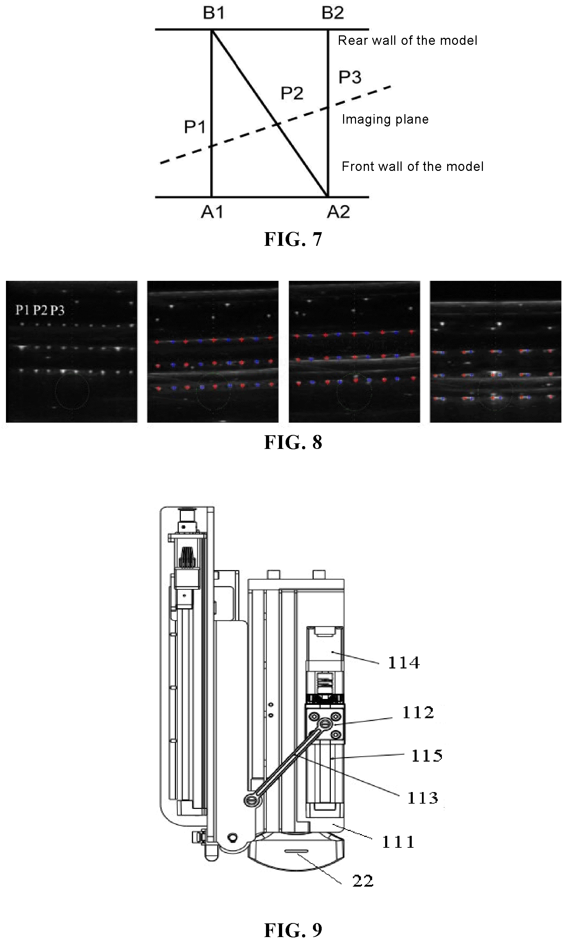

As shown in FIGS. 6 to 8, in the present embodiment, the calibration module comprises fine wires threaded through a perforated model. Two parallel wires and one oblique wire form an "N"-shaped structure. Each "N"-shaped structure is regarded as a basic unit, and the ultrasound imaging plane intersects each unit at three points (P1, P2, and P3). The four endpoints of each "N" shape are A1, A2, B2, and B1, respectively.

In the present embodiment, obtaining the first transformation matrix $T P F$ between the base coordinate system {F} of the surgical operation device and the coordinate system {P} of the calibration module mounted on the base of the surgical operation device comprises: obtaining the base coordinate system {F} of the surgical operation device and a Marker coordinate system {M} of a visual Marker mounted on the surgical operation device, so as to obtain a third transformation matrix $T M F$ between the Marker coordinate system and the base coordinate system of the surgical operation device; obtaining the coordinate system {P} of the calibration module mounted on the base of the surgical operation device, so as to obtain a fourth transformation matrix $T P M$ between the coordinate system {P} of the calibration module and the Marker coordinate system {M}; obtaining, based on the third transformation matrix $T M F$ and the fourth transformation matrix $T P M$, the first transformation matrix $T P F$ between the coordinate system {P} of the calibration module and the base coordinate system {F} of the surgical operation device. Such a method facilitates computation of the first transformation matrix, thereby enabling subsequent calculation of the second transformation matrix. Specifically, the first transformation matrix is typically precomputed and calibrated prior to factory shipment, thereby enabling rapid determination of the second transformation matrix during subsequent use.

Specifically, mapping the features of the ultrasound image to corresponding actual three-dimensional features to obtain a second transformation matrix $T l F$ between the coordinate system {I} of the ultrasound image and the base coordinate system {F} of the surgical operation device, and determining, based on the second transformation matrix $T l F$, positional deviations of the base of the surgical operation device relative to the ultrasound image space and the physical space, comprises:
setting pixel scaling factors of the ultrasound image as Sx and Sy;
setting **(*u*,*v*)** as pixel coordinates in the ultrasound image coordinate system {I}, and setting **(*x*, *y*, *z*)** as three-dimensional coordinates corresponding to **(*u*, *v*)** in the coordinate system {P} of the calibration module:
   establishing the following formula: $\left[\begin{matrix}x \\ y \\ z \\ 1\end{matrix}\right] = T - 1 P F T I F \left[\begin{matrix}{s}_{x} u \\ {s}_{y} ν \\ 0 \\ 1\end{matrix}\right] .$

Such a method facilitates establishment of a transformation relationship between the ultrasound image coordinate system {I} and the coordinate system of the calibration module, thereby enabling more accurate determination of the corresponding second transformation matrix.

In the present embodiment, the calibration module is an N-wire module; mapping the features of the ultrasound image to corresponding actual three-dimensional features to obtain a second transformation matrix $T I F$ between the coordinate system {I} of the ultrasound image and the base coordinate system {F} of the surgical operation device, and determining, based on the second transformation matrix $T I F$, positional deviations of the base of the surgical operation device relative to the ultrasound image space and the physical space, further comprises: designating three intersection points between the ultrasound imaging plane and the three line segments of each N-shaped structure of the N-wire module as point P1, point P2, and point P3, respectively, and obtaining coordinates ***P*_{*A*1}, *P*_{A2,}*P*_{*B*1}, *P*_{B2}** of four endpoints A1, A2, B1, and B2 of each N-shaped structure in the coordinate system {P} of the calibration module, wherein the point P1, the point P2, and the point P3 are respectively intersection points between the ultrasound imaging plane and a line on a first side of the N-shape, a middle line of the N-shape, and a line on a second side of the N-shape;
extracting pixel coordinates ***p*₁,*p*₂,*p*₃** of the point P1, the point P2, and the point P3 in the coordinate system {I} of the ultrasound image, respectively;
calculating coordinates ***P*₂** of the point P2 in the coordinate system {P} of the calibration module according to a similar-triangle principle,
wherein ${P}_{Z} = \left(1-λ\right) {P}_{B 1} + λ {P}_{AZ} ; λ = \frac{\left‖{p}_{1}-{p}_{2}\right‖}{\left‖{p}_{1}-{p}_{3}\right‖} ;$
wherein two-dimensional pixel coordinates and three-dimensional coordinates of the point P2 in the model coordinate system {P} are respectively represented by ***pᵢ*** = **[*uᵢ*,*vᵢ*]^{T}** and ***Pᵢ*, *i* = 1, ···,*K*;** wherein K denotes a quantity of N-wires;
setting R and t respectively as a rotation matrix and a translation vector of the first transformation matrix $T P F$, such that the following relationship is derived:
   ***sₓuᵢr*₁** + ***s_{y}v*ᵢ*r*₂** + ***t* = *yᵢ*.**
wherein, ***r***₁ and ***r***₂ are first two columns of a rotation component ***R*** of the second transformation matrix $T I F$, and ***t*** is a translation vector of $T I F$, ***y**ᵢ = **R P**ᵢ* + ***t***; let ***a***₁ = *sₓ**r***₁,***a***₂ = *s_{y}**r***₂, $x = {\left[{a}_{1}^{T}, {a}_{2}^{T}, {t}^{T}\right]}^{T}$, and the above formula is rewritten in matrix form as: ${A}_{i} x = {y}_{i}$
where: ${A}_{i} = \left[\begin{matrix}{u}_{i} & 0 & 0 & {ν}_{i} & 0 & 0 & 1 & 0 & 0 \\ 0 & {u}_{i} & 0 & 0 & {ν}_{i} & 0 & 0 & 1 & 0 \\ 0 & 0 & {u}_{i} & 0 & 0 & {ν}_{i} & 0 & 0 & 1\end{matrix}\right] ,$
stacking all ***Aᵢ*** and ***yᵢ*** (*i* = **1, ···,*K***) row-wise to form a final linear equation ***Ax*** = ***y*,** a least-squares solution thereof being ***x*** = **(*A^{T}A*)⁻¹*A^{T}y*;**
after ***x*** is determined, ***a***₁, ***a***₂, and ***t*** are obtained accordingly;
after calculation, ***sₓ*** = ∥***a*₁**∥, ***s_{y}*** = ∥***a*₂**∥, ***r*₁ = *a*₁/*sₓ***, ***r*₂** = ***a*₂/*s_{y}***, and ***r*₃** = ***r*₁** × ***r*₂** are obtained so as to calculate the second transformation matrix.

Such a method facilitates calculation of the second transformation matrix, thereby enabling accurate determination of the positional deviation between the base of the surgical operation device and the physical space of the ultrasound probe.

Specifically, after obtaining ***sₓ*** = ∥***a*₁**∥, ***s_{y}*** = ∥***a*₂**∥, ***r*₁** = ***a*₁/*sₓ***, ***r*₂** = ***a*₂/*s_{y},*** and ***r*₃** = ***r*₁** × ***r*₂** through calculation so as to calculate the second transformation matrix, the calibration method further comprises:
calculating a root-mean-square error $RMSE = \sqrt{\frac{1}{n} {\sum }_{i = 1}^{n} {\left‖{T}_{FI}P i I-P i F\right‖}^{2}}$;
defining outliers according to {∪***P**ᵢ* | ∥***T**_{FP} ^{P}**P**ᵢ* - *^{F}**P**ᵢ*∥ *> RMSE*}, and removing outliers satisfying the outlier definition formula from original data;
recalculating *sₓ* and *s_{y},* and recalculating to obtain the second transformation matrix ***T**_{FP}.* Such a method improves the accuracy of solving the second transformation matrix, thereby enabling more accurate determination of the positional deviation between the base of the surgical operation device and the physical space of the ultrasound probe, and further improving operational precision of the surgical operation device.

Specifically, the ultrasound probe calibration in the present patent determines the coordinate transformation relationship T_FI between the robot end-effector flange coordinate system {F} and the two-dimensional ultrasound image coordinate system {I}. Once the ultrasound probe is mounted on the robot end-effector and the imaging parameters of the ultrasound probe are fixed, the coordinate transformation relationship T_FI remains constant. The coordinate transformation relationship T_FI can be used to map pixels in the ultrasound image to points in three-dimensional space, and to correlate the mapped points with the position of the puncture needle in the puncture mechanism.

As shown in FIG. 2, a schematic diagram is illustrated showing the positional relationship among four coordinate systems of the surgical operation device. The four coordinate systems include: a Marker coordinate system {M} of a visual Marker, a calibration module coordinate system {P}, an ultrasound image coordinate system {I}, and a base coordinate system {F} of the surgical operation device.

Specifically, in the present embodiment, ultrasound probe calibration includes: mounting a visual Marker at the end-effector of the surgical operation device (also understood as the robotic arm of a surgical robot), and performing hand-eye calibration to obtain the transformation relationship T_FM between the Marker and the end-effector; mounting an N-wire calibration module at the robotic arm end-effector via a quick-mount structure, and using a probe to detect grooves on the model; registering the N-wire calibration module in the Marker coordinate system {M}, and transforming the N-wire calibration module into the robot end-effector coordinate system {F} using T_FM to obtain T_FP. As long as the mounting relationship between the N-wire calibration module and the robot end-effector remains unchanged, calibration is required only once, thereby significantly facilitating clinical calibration procedures. By performing ultrasound imaging of the N-wire calibration module having known geometric dimensions, features in ultrasound images are extracted and mapped to corresponding three-dimensional features. Finally, a least-squares optimization algorithm is employed to minimize a feature-matching loss function, thereby estimating an unknown transformation relationship.

Specifically, the base coordinate system of the surgical operation device may also be referred to as the robot end-effector coordinate system {F}; the Marker coordinate system of the visual Marker on the surgical operation device may also be referred to as the Marker coordinate system {M} on the robot end-effector; the ultrasound image coordinate system may also be referred to as the image coordinate system {I}; and the calibration module coordinate system may also be referred to as the calibration model coordinate system {P}. The transformation matrix ***T***_{FI} between the image coordinate system and the robot end-effector coordinate system is an unknown parameter, which, together with ultrasound image pixel spacing parameters *sₓ* and *s_{y}*, constitutes a set of parameters to be solved. Let (*u*,*v*) denote pixel coordinates in the image coordinate system, and the corresponding three-dimensional coordinates (*x*,*y*,*z*) in the model coordinate system are expressed as: $\left[\begin{matrix}x \\ y \\ z \\ 1\end{matrix}\right] = {T}_{FP}^{- 1} {T}_{FI} = \left[\begin{matrix}{s}_{x} u \\ {s}_{y} ν \\ 0 \\ 1\end{matrix}\right]$

The N-wire calibration module comprises an "N"-shaped structure formed by two parallel wires and one oblique wire. Each "N"-shaped structure is regarded as a basic unit, and the ultrasound imaging plane intersects each unit at three points, denoted as P1, P2, and P3. The endpoints A1, A2, B1, and B2 of the N-wire are known in the model coordinate system and are respectively denoted as vectors ***P***_{A1},***P***_{*A*2},***P***_{*B*1},***P***_{*B*2} ∈□ ³. In the present embodiment, image processing algorithms including adaptive threshold segmentation, morphological processing, connected-component analysis, and clustering are used to extract the pixel coordinates of P1, P2, and P3 in the ultrasound image, as shown in the figure below, denoted respectively as ***p***₁, ***p***₂, ***p***₃ ∈□ ².

According to a similar-triangle principle, coordinates ***P***₂ ∈□ ³ of the point P2 in the model coordinate system can be calculated: ${P}_{2} = \left(1-λ\right) {P}_{B 1} + λ {P}_{A 2}$ $λ = \frac{\left‖{p}_{1}-{p}_{2}\right‖}{\left‖{p}_{1}-{p}_{3}\right‖} ;$

Based on the N-wire calibration, a top-view correspondence between the N-wire model and the intersection points of the N-wire with the ultrasound image is obtained from the actual ultrasound image.

Specifically, the calibration model comprises three rows, each row including four "N" units. Red points represent P1 and/or P3, and blue points represent P2. A single ultrasound image can yield 12 P2 points, and the corresponding two-dimensional pixel coordinates and three-dimensional coordinates in the model coordinate system are respectively represented by ***p**ᵢ* = [*uᵢ*,*vᵢ*]^{T} and ***Pᵢ***, *i* = 1,···,12. ***P**ᵢ* can be calculated according to the above formula. Let ***R,t*** denote the rotation matrix and translation vector of ***T**_{FP} ,* respectively. From the above expression, the following is obtained: ${s}_{x} {u}_{i} {r}_{1} + {s}_{y} {ν}_{i} {r}_{2} + t = {y}_{i} ;$
where ***r***₁ and ***r***₂ are first two columns of a rotation component ***R*** of the unknown matrix ***T**_{FI}*, and ***t*** is a translation vector of ***T**_{FI}*, ***y**ᵢ = **R P**ᵢ +**t***. Let ***a*₁** = *sₓ**r***₁*,**a***₂ = *s_{y}**r***₂, $x = {\left[{a}_{1}^{T}, {a}_{2}^{T}, {t}^{T}\right]}^{T}$, and the above formula is rewritten in matrix form as: ${A}_{i} x = {y}_{i} ;$
where: ${A}_{i} = \left[\begin{matrix}{u}_{i} & 0 & 0 & {ν}_{i} & 0 & 0 & 1 & 0 & 0 \\ 0 & {u}_{i} & 0 & 0 & {ν}_{i} & 0 & 0 & 1 & 0 \\ 0 & 0 & {u}_{i} & 0 & 0 & {ν}_{i} & 0 & 0 & 1\end{matrix}\right] ,$

Stacking all ***A**ᵢ* and ***y**ᵢ* (*i* = 1,··· ,12) row-wise yields a final linear system ***Ax*=*y***, and the least-squares solution thereof is: ***x*** =(***A***^{T}***A***)⁻¹ ***A***^{T}***y***. Once *x* is solved, ***a***₁, ***a***₂, and ***t*** are obtained. Further, the following parameters are derived: *sₓ* =∥***a***₁∥, *s_{y}* = ∥***a***₂∥, ***r***₁ =***a***₁ / *sₓ*, ***r***₂ *= **a***₂ / *s_{y}*, and ***r***₃ =***r***₁×***r***₂. Thus, all unknown transformation parameters have been determined.

To further improve accuracy, the results may be further optimized. Since the acquired data may contain errors arising from experimental operations or feature extraction, outliers are removed based on the initial solution ***T**_{FI}*, and a second calculation is performed. The root mean square error is defined as: $RMSE = \sqrt{\frac{1}{n} {\sum }_{i = 1}^{n} {\left‖{{T}_{FI}}^{I}{P}_{i}-P i F\right‖}^{2}} .$

Specifically, outliers are defined as {∪***P**ᵢ* | ∥***T**_{FP} ^{P}**P**ᵢ - ^{F}**P**ᵢ*∥ *> RMSE*}. After removing the outliers from the original data, *sₓ*, *s_{y}*, and ***T**_{FP}* are recalculated, thereby improving computational accuracy.

Embodiment 2 of the present invention provides a calibration device for a surgical operation device, the calibration device comprising: an acquisition module, an ultrasound imaging module, and a computation module. The acquisition module is configured to obtain a first transformation matrix $T P F$ between the base coordinate system {F} of the surgical operation device and the coordinate system {P} of a calibration module mounted on the base of the surgical operation device. The ultrasound imaging module is configured to perform ultrasound imaging of the calibration module using an ultrasound probe and to extract features from the ultrasound image. The computation module is configured to map features of the ultrasound image to corresponding actual three-dimensional features so as to obtain a second transformation matrix $T I F$ between the ultrasound image coordinate system {I} and the base coordinate system {F} of the surgical operation device, and determine, based on the second transformation matrix $T I F$, a positional deviation of the base of the surgical operation device relative to the physical space of the ultrasound probe.

Specifically, the computation module comprises a first setting module, a second setting module, and a formula establishment module. The first setting module is configured to set pixel scaling factors of the ultrasound image as Sx and Sy. The second setting module is configured to set **(*u*,*v*)** as pixel coordinates in the ultrasound image coordinate system {I}, and to set **(*x*, *y*, *z*)** as three-dimensional coordinates corresponding to **(*u*,*v*)** in the calibration module coordinate system {P}.

The formula establishment module is configured to establish the following formula: $\left[\begin{matrix}x \\ y \\ z \\ 1\end{matrix}\right] = T - 1 P F T I F \left[\begin{matrix}{s}_{x} u \\ {s}_{y} v \\ 0 \\ 1\end{matrix}\right] .$

Embodiment 3 of the present invention provides a non-volatile storage medium, wherein the non-volatile storage medium stores a plurality of instructions, the instructions being configured to be loaded and executed by a processor so as to cause the processor to perform the calibration method described above.

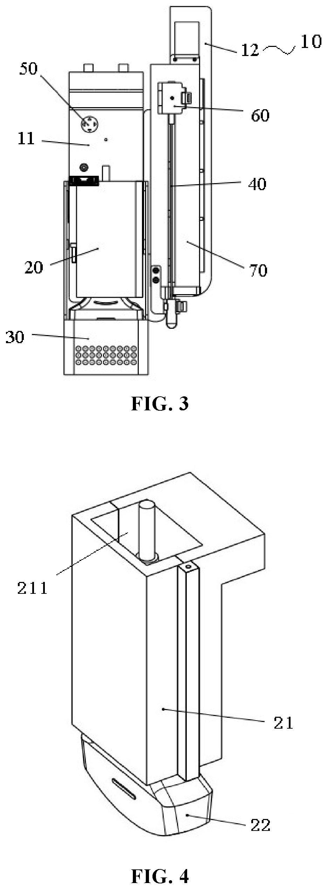

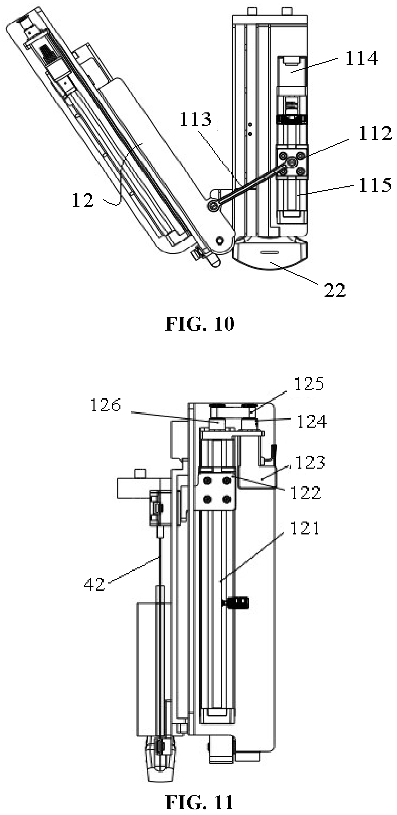

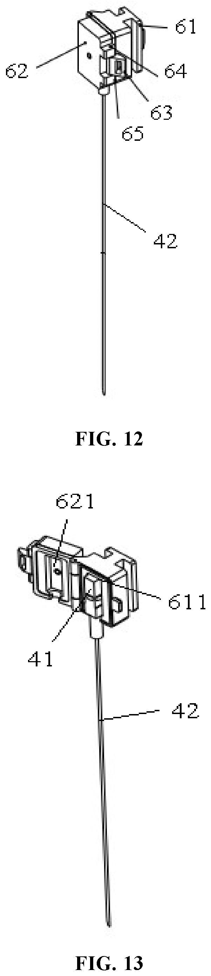

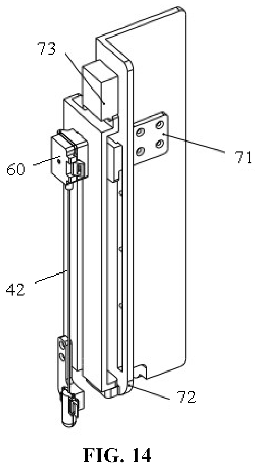

As shown in FIGS. 3-5 and 9-14, Embodiment 4 of the present invention provides a surgical operation device configured for use with the calibration method described above. The surgical operation device comprises an operation member 10, an ultrasound probe, and a calibration module 30. The operation member 10 is movably disposed; the ultrasound probe is mounted on the operation member 10; and the calibration module 30 is detachably mounted on the operation member 10, wherein the calibration module 30 comprises a calibration wire 31 having a plurality of N-shaped structures, and the calibration wire 31 is disposed opposite the ultrasound probe. With such a structural arrangement, the calibration module 30 is mounted on the operation member 10 before a surgical puncture procedure is performed by the surgical operation device. Calibration of the ultrasound probe is performed through cooperation between the ultrasound probe and the calibration module 30. After completion of the calibration, the calibration module 30 is detachable from the operation member (10), thereby preventing interference of the calibration module (30) with the surgical operation device during a puncture process and ensuring operational accuracy of the surgical operation device.

In the present embodiment, the calibration module 30 further comprises a mounting body 32, which is detachably mounted on the operation member 10. The mounting body 32 is provided with a first mounting side wall and a second mounting side wall oppositely disposed. The first mounting side wall is provided with a plurality of first threading holes, and the second mounting side wall is provided with a plurality of second threading holes. The calibration wire 31 is threaded back and forth between the plurality of first threading holes and the plurality of second threading holes to form a plurality of N-shaped structures. Such a structural arrangement facilitates threading of the calibration wire 31 to form the plurality of N-shaped structures, thereby facilitating subsequent calibration operations.

Specifically, the calibration module 30 further comprises a first detachable arm 33 and a second detachable arm 34 spaced apart from each other. The first detachable arm 33 and the second detachable arm 34 are oppositely arranged on two sides of the mounting body 32. The first detachable arm 33 and the second detachable arm 34 are respectively configured to be snap-fitted to two sides of the ultrasound probe and detachably connected thereto, and the mounting body 32 is positioned below a detection head of the ultrasound probe. Such an arrangement facilitates installation and removal of the calibration module 30.

Specifically, the first detachable arm 33 is provided with two first connecting arms spaced apart and arranged parallel to each other, and the second detachable arm 34 is provided with two second connecting arms spaced apart and arranged parallel to each other. The two first connecting arms and the two second connecting arms are oppositely arranged. Each of the two first connecting arms is provided with a first dismounting hole, and each of the two second connecting arms is provided with a second dismounting hole. The first dismounting holes and the second dismounting holes are configured to cooperate with the ultrasound probe to achieve installation and removal of the calibration module 30.

In the present embodiment, the ultrasound probe comprises a mounting member 21 and a probe portion 22 connected to each other. The mounting member 21 is provided with a mounting channel 211. The probe portion 22 is mounted on the mounting member 21, wherein a portion of the probe portion 22 is within the mounting channel 211, and another portion of the probe portion 22 extends out of the mounting channel 211. The mounting member 21 comprises a fixed end and an openable end spaced apart from each other, wherein the openable end is rotatably disposed relative to the fixed end so as to enable adaptive adjustment of an angle of the probe portion 22. Specifically, the probe portion 22 may be a B-mode ultrasound probe portion.

The contact surfaces of the fixed end and the openable end coincide with a central plane of the probe portion 22, thereby ensuring that the rotational plane coincides with the central plane of the probe portion (22), and ensuring that the mounting member (21) avoids a situation in which a relative positional deviation occurs between the probe portion (22) and the mounting member (21) during rotation.

Specifically, the openable end is configured to open and close via a rotational movement. In order to ensure that the B-mode ultrasound probe and the puncture needle are coplanar, the contact surfaces of the fixed end and the openable end are arranged in a plane in which the puncture needle is positioned, thereby ensuring coplanarity between the B-mode ultrasound probe and the puncture needle and effectively improving puncture accuracy.

Specifically, the surgical operation device further comprises a puncture needle 40, wherein the puncture needle 40 is positioned on a central plane of the probe portion 22. Such a structural configuration ensures coplanarity between the probe portion 22 and the puncture needle 40, thereby enabling more accurate determination of the position of the puncture needle 40 via the probe portion 22 and improving positional accuracy.

In the present embodiment, the surgical operation device further comprises a puncture needle 40. The operation member 10 comprises an angle control module 11 and a depth control module 12. A drive portion of the angle control module 11 is rotatably disposed relative to a body portion 111 of the angle control module 11, and an ultrasound probe 20 is mounted on the body portion 111 of the angle control module 11. The drive portion of the angle control module 11 is drivingly connected to the depth control module 12. A drive end of the depth control module 12 is movably disposed along a preset direction, and is drivingly connected to the puncture needle 40. With such a structural arrangement, the rotation angle and insertion depth of the puncture needle 40 can be conveniently controlled, thereby facilitating guidance of the puncture needle 40 to a lesion site and improving puncture accuracy at the lesion site. Specifically, the angle control module 11 enables the puncture needle 40 to rotate about an axis, thereby automatically adjusting an angle of the puncture needle 40.

Specifically, the angle control module 11 comprises a slider 112 and a connecting rod 113. The slider 112 is movably disposed on a body portion 111 of the angle control module 11 along a preset direction. The connecting rod 113 forms a drive portion of the angle control module 11, wherein one end of the connecting rod 113 is hinged to the slider 112, and the other end thereof is hinged to the depth control module 12. Such a configuration has a simple structure and stable transmission performance, thereby enabling stable angular deflection.

The angle control module 11 further comprises a motor 114 and a lead screw guide assembly 115. The motor 114 drives a lead screw of the lead screw guide assembly 115 to rotate. The slider 112 is mounted on the lead screw and is threadedly engaged therewith, such that the slider 112 is driven to move by the lead screw guide assembly 115.

Specifically, the depth control module 12 comprises a lead screw guide rail module 121 and a drive block 122. The lead screw guide rail module 121 is rotatably arranged. The drive block 122 is mounted on the lead screw guide rail module 121 and is threadedly engaged therewith, such that rotation of the lead screw guide rail module 121 drives the drive block 122 to move along a preset direction. Such a configuration enables stable driving of the drive block 122 in the preset direction, thereby improving the driving stability of the drive block 122.

The depth control module 12 further comprises a drive motor 123, a driving timing pulley 124, a timing belt 125, a driven timing pulley 126, the lead screw guide rail module 121, and the drive block 122. The drive motor 123 drives the driving timing pulley 124 to rotate, thereby driving the timing belt 125, which in turn drives rotation of the driven timing pulley 126. Rotation of the driven timing pulley 126 further drives rotation of a lead screw, which drives the drive block 122 to move linearly in a vertical direction. The slider 112 further drives movement of a quick-release structure for the puncture needle 40, thereby driving linear movement of the puncture needle 40 such that a tip of the puncture needle 40 reaches a lesion site.

In the present embodiment, the surgical operation device further comprises a micro-adjustment control module 50, which is mounted on the operation member 10 and is configured to adjust the angle control module 11 and the depth control module 12. With such a configuration, the micro-adjustment control module 50 is used to compensate for positional deviation of the lesion during the puncture process, and the physician may switch to a manual adjustment mode to finely adjust the angle or depth of the puncture needle 40. The micro-adjustment control module 50 enables automated control. Specifically, the micro-adjustment control module 50 may be implemented using a programmable logic controller (PLC) or a microcontroller. Remote control may also be adopted, enabling the physician to adjust the puncture needle 40 via the micro-adjustment control module 50 outside the operating room, thereby reducing radiation exposure to the physician from medical equipment in the operating room.

Specifically, the depth control module 12 may be remotely controlled, enabling the physician to adjust the puncture needle 40 outside the operating room via the micro-adjustment control module 50. The angle control module 11 may also be remotely controlled, enabling the physician to adjust the puncture needle 40 outside the operating room via the micro-adjustment control module 50.

Specifically, the operation member 10 has an initial state and an operational state for puncture. When the operation member (10) is in an initial state, the angle control module (11) and the depth control module (12) are closely fitted to each other, thereby reducing an overall spatial footprint of the operation member (10) and improving structural compactness thereof.

In the present embodiment, the surgical operation device further comprises a puncture needle 40 and a quick-release structure 60. The puncture needle 40 is mounted on the quick-release structure 60, and the quick-release structure 60 is detachably mounted on the operation member 10. Such a structural arrangement facilitates rapid manipulation of the puncture needle 40. Specifically, after the puncture needle 40 accurately reaches the lesion, the physician may detach the quick-release structure 60 so as to separate the puncture needle 40 from the operation member 10, allowing the puncture needle 40 to remain in the patient's body and facilitating subsequent surgical procedures.

Specifically, the quick-release structure 60 comprises a base 61 and a quick-release plate 62, wherein the quick-release plate 62 is movably disposed on the base 61 so as to move between a clamping position, in which the quick-release plate 62 is disposed opposite at least a portion of the base 61 and clamps the puncture needle 40, and a release position, in which the quick-release plate 62 is displaced away from the base 61, thereby facilitating clamping or removal of the puncture needle 40 and further improving operational convenience.

Specifically, the quick-release plate 62 is rotatably mounted on the base 61.

In the present embodiment, the base 61 is provided with a first snap-fit structure 63, and the quick-release plate 62 is provided with a second snap-fit structure 64 adapted to the first snap-fit structure 63. When the quick-release plate 62 is in the clamping position, the first snap-fit structure 63 is snap-engaged with the second snap-fit structure 64. The quick-release structure 60 further comprises an elastic member 65 disposed on the first snap-fit structure 63 or the second snap-fit structure 64, the elastic member 65 being positioned between the first snap-fit structure 63 and the second snap-fit structure 64. Such a configuration helps ensure stable clamping of the puncture needle 40. At least one of the first snap-fit structure 63 and the second snap-fit structure 64 is rotatably disposed. The elastic member 65 may be an elastic silicone pad configured to ensure stable engagement between the first snap-fit structure 63 and the second snap-fit structure 64, thereby preventing disengagement during a surgical procedure.

Specifically, the puncture needle 40 comprises a mounting portion 41 and a needle body 42. The base 61 is provided with a first mounting groove 611 adapted to the mounting portion 41, and the quick-release plate 62 is provided with a second mounting groove 621 adapted to the mounting portion 41. When the quick-release plate 62 moves to the clamping position, the first mounting groove 611 and the second mounting groove 621 are joined together to form an enclosure around the periphery of the mounting portion 41, and the needle body 42 extends out of the quick-release structure 60. Such a configuration facilitates improved mounting of the mounting portion 41 and enhances the mounting stability of the puncture needle 40.

In the present embodiment, the surgical operation device further comprises an isolation plate 70, which is detachably mounted on the operation member 10, and the puncture needle 40 is detachably mounted on a side of the isolation plate 70 away from the operation member 10. Such a structural configuration enables isolation between the operation member 10 and the puncture needle 40, thereby separating the sterile field and the non-sterile field, ensuring a sterile surgical environment and preventing bacteria from the operation member 10 from entering the puncture needle 40, so as to provide improved aseptic protection for the puncture needle 40. Specifically, the isolation plate 70 is a sterile isolation plate and is a consumable component; one isolation plate 70 corresponds to one puncture needle 40, and the isolation plate 70 must be replaced when the puncture needle 40 is replaced.

Specifically, a sterile membrane is provided on the isolation plate 70.

Specifically, the isolation plate 70 is detachably mounted on the operation member 10, and the puncture needle 40 is detachably mounted on the isolation plate 70 via a quick-release structure 60. Specifically, the isolation plate 70 is provided with mounting rails, and the quick-release structure 60 is provided with mounting blocks adapted to the mounting rails. The quick-release structure 60 is mounted on the isolation plate 70 through cooperation between the mounting blocks and the mounting rails.

Specifically, an adapter plate 71 is provided on the isolation plate 70, and the adapter plate 71 is coupled to the drive block 122 via a plug-in structure 72. The adapter plate 71 is driven by the drive block 122 of the depth control module 12 to move linearly in a vertical direction, thereby driving the quick-release assembly to move linearly in a vertical direction, and further driving the puncture needle to move linearly in a vertical direction. The sterile membrane isolation plate 70 is engaged with the base 61 via a pin-and-socket structure, and assembly and disassembly between the two can be further achieved via a pressing structure 73.

Specifically, a connecting pin is provided at an end of the sterile membrane isolation plate 70, and the connecting pin is insertable into a corresponding socket hole in the base 61.

Specifically, the pressing structure 73 is a press-type quick-release structure and comprises a spring-loaded retractable stopper. When the sterile membrane isolation plate 70 contacts the spring stopper, the spring stopper is pushed open. when the sterile membrane isolation plate 70 is fully pressed into position, the spring stopper returns to its original position to achieve quick assembly. During disassembly, pressing the side causes the spring stopper to retract, thereby enabling quick release.

Specifically, the lesion site is obtained through ultrasound imaging, and a puncture path is planned by an algorithm, wherein the puncture path is defined as a line connecting the needle insertion starting point and the lesion. The angle control module 11 is further controlled to rotate the puncture needle 40 onto the puncture path, and the depth control module 12 is further controlled, thereby enabling fully automated puncture. During the fully automated puncture process, pixel information of the ultrasound image is converted into dimensional information via an algorithm, and the dimensional information is further converted into rotational angle information of the motor 114. A clamping module using the imaging central plane of the ultrasound probe 20 as a reference ensures collinearity between the ultrasound probe 20 and the puncture needle 40. The calibration module 30 compensates for positional deviation of the ultrasound probe 20 on the imaging plane, thereby further improving the accuracy of ultrasound image acquisition.

Specifically, puncture is a dynamic process, and the lesion site may shift during puncture due to factors such as patient respiration or changes in body posture. To further ensure puncture accuracy, the present invention further provides a micro-adjustment device. During advancement of the puncture needle 40, the ultrasound probe monitors in real time the positions of the puncture needle 40 and the lesion. If a positional change of the lesion is detected, the angle of the puncture needle 40 is adjusted via a micro-adjustment button so that the puncture needle 40 is aligned with the lesion, thereby further improving puncture accuracy.

From the above description, it can be seen that the embodiments of the present invention achieve the following technical effects: The calibration module compensates for clamping errors of the ultrasound probe. The angle control module adopts a structurally stable lead screw mechanism and a crank-slider mechanism, enabling automatic angular positioning and alignment with a pre-planned puncture path. The depth control module adopts a structurally stable lead screw mechanism, providing good dimensional stability of key assemblies and enabling automatic needle advancement. The micro-adjustment controller enables real-time observation of B-mode ultrasound images to compensate for lesion displacement. The physician may perform manual control or remote operation to enable micro-adjustment of the angle control module and the depth control module, thereby achieving precise control of the puncture needle. The puncture needle quick-release structure enables rapid detachment of the puncture needle from the operation member after completion of the puncture procedure, when the entire puncture device is to be withdrawn while leaving only the puncture needle within the patient. Such a configuration significantly reduces surgical time and improves procedural efficiency. The consumable assembly comprising a sterile membrane, an isolation plate, the puncture quick-release structure, and the puncture needle adopts a plug-and-press configuration, enabling rapid disassembly and thereby reducing both preoperative preparation time and postoperative removal time. By precisely controlling the puncture angle and position, the puncture needle is guided to a phantom lesion site. The system further simulates intraoperative lesion displacement, and real-time lesion tracking is achieved through micro-adjustment of the puncture angle and puncture depth, thereby further improving puncture accuracy.

It should be noted that the terminology used herein is for the purpose of describing specific embodiments only and is not intended to limit the exemplary embodiments of the present application. As used herein, unless the context clearly dictates otherwise, the singular forms are intended to include the plural forms. Furthermore, it should be understood that when the terms "comprise" and/or "include" are used in the present specification, such terms indicate the presence of features, steps, operations, devices, components, and/or combinations thereof.

Unless otherwise specifically stated, the relative arrangements of components and steps, numerical expressions, and values set forth in these embodiments do not limit the scope of the present application. It should also be understood that, for clarity of illustration, the dimensions of the various components shown in the drawings are not necessarily drawn to actual scale. Techniques, methods, and equipment known to those of ordinary skill in the art may not be described in detail; however, where appropriate, such techniques, methods, and equipment should be considered part of the specification. In all examples shown and discussed herein, any specific values should be interpreted as merely illustrative and not as limiting. Accordingly, other examples of the exemplary embodiments may have different values. It should be noted that like reference numerals and letters in the accompanying drawings denote like elements; therefore, once an element is defined in one drawing, it need not be further described in subsequent drawings.

In the description of the present application, it should be understood that directional terms such as "front", "rear", "upper", "lower", "left", and "right", as well as "horizontal", "vertical", "top", and "bottom", refer generally to the orientations or positional relationships shown in the drawings. Such terms are used solely for convenience of description and to simplify the description of the present application. Unless otherwise explicitly stated, the directional terms do not indicate or imply that the device or elements referred to are required to have a specific orientation or to be constructed or operated in a specific orientation. Accordingly, such terms shall not be construed as limiting the scope of the present application. The terms "inner" and "outer" refer to positions relative to the interior and exterior of the outline of each respective component.

For clarity of description, spatial relative terms such as "above", "on top of", "on the upper surface of", and "upper", may be used herein to describe the spatial positional relationship between one device or feature as shown in the drawings and other devices or features. It should be understood that such spatially relative terms are intended to encompass different orientations of the device during use or operation in addition to the orientation illustrated in the drawings. For example, if a device in the drawings is inverted, an element described as being "above" or "on top of" another device or structure would then be positioned "below" or "under" that other device or structure. Accordingly, the exemplary term "above" may encompass both "above" and "below" orientations. The device may also be positioned in other orientations (e.g., rotated by 90 degrees or otherwise oriented), and the spatial relative terms used herein should be interpreted accordingly.

In addition, it should be noted that the use of terms such as "first" and "second" to define components is solely for the purpose of distinguishing corresponding components. Unless otherwise stated, such terms have no special meaning and should not be construed as limiting the scope of protection of the present application.

The above description is only of preferred embodiments of the present invention and is not intended to limit the present invention. Those skilled in the art will recognize that various modifications and variations may be made to the present invention. Any modifications, equivalent substitutions, improvements, and the like made within the spirit and principle of the present invention shall fall within the scope of protection of the present invention.

## Claims

1. A calibration method for an ultrasound probe of a surgical operation device, **characterized in that** it comprises:
obtaining a first transformation matrix $T P F$ between a base coordinate system {F} of the surgical operation device and a coordinate system {P} of a calibration module mounted on a base of the surgical operation device;
performing ultrasound imaging of the calibration module using the ultrasound probe, and extracting features from an ultrasound image;
mapping the features of the ultrasound image to corresponding actual three-dimensional features to obtain a second transformation matrix $T I F$ between a coordinate system {I} of the ultrasound image and the base coordinate system {F} of the surgical operation device; and determining, based on the second transformation matrix $T I F$, a positional deviation between the base of the surgical operation device and a physical space of the ultrasound probe.

2. The calibration method according to claim 1, **characterized in that** obtaining the first transformation matrix $T P F$ between the base coordinate system {F} of the surgical operation device and the coordinate system {P} of the calibration module mounted on the base of the surgical operation device comprises:
obtaining the base coordinate system {F} of the surgical operation device and a Marker coordinate system {M} of a visual Marker mounted on the surgical operation device, so as to obtain a third transformation matrix $T M F$ between the Marker coordinate system {M} and the base coordinate system {F} of the surgical operation device;
obtaining the coordinate system {P} of the calibration module mounted on the base of the surgical operation device, so as to obtain a fourth transformation matrix $T P M$ between the coordinate system {P} of the calibration module and the Marker coordinate system {M}; and
obtaining the first transformation matrix $T P F$ between the coordinate system {P} of the calibration module and the base coordinate system {F} of the surgical operation device based on the third transformation matrix $T M F$ and the fourth transformation matrix $T P M$*.*

3. The calibration method according to claim 1, **characterized in that** mapping features of the ultrasound image to corresponding actual three-dimensional features so as to obtain the second transformation matrix $T I F$ between the coordinate system {I} of the ultrasound image and the base coordinate system {F} of the surgical operation device, and determining, based on the second transformation matrix $T I F$, positional deviations of the base of the surgical operation device relative to the ultrasound image space and the physical space, comprises:
setting pixel scaling factors of the ultrasound image as Sx and Sy;
setting **(*u*,*v*)** as pixel coordinates in the coordinate system {I} of the ultrasound image, and setting **(*x*, *y*, *z*)** as three-dimensional coordinates corresponding to **(*u*,*v*)** in the coordinate system {P} of the calibration module; and
establishing the following formula: $\left[\begin{matrix}x \\ y \\ z \\ 1\end{matrix}\right] = T - 1 P F T l F \left[\begin{matrix}{s}_{x} u \\ {s}_{y} v \\ 0 \\ 1\end{matrix}\right] .$

4. The calibration method according to claim 3, **characterized in that** the calibration module is an N-wire module; mapping the features of the ultrasound image to corresponding actual three-dimensional features to obtain the second transformation matrix $T I F$ between the coordinate system {I} of the ultrasound image and the base coordinate system {F} of the surgical operation device, and determining, based on the second transformation matrix $T I F$, positional deviations of the base of the surgical operation device relative to the ultrasound image space and the physical space, further comprises:
designating three intersection points between the ultrasound imaging plane and the three line segments of each N-shaped structure of the N-wire module as point P1, point P2, and point P3, respectively, and obtaining coordinates ***P*_{*A*1}, *P*_{A2},*P*_{*B*1}, *P*_{*B*2}** of four endpoints A1, A2, B1, and B2 of each N-shaped structure in the coordinate system {P} of the calibration module, wherein the point P1, the point P2, and the point P3 are respectively intersection points between the ultrasound imaging plane and a line on a first side of the N-shape, a middle line of the N-shape, and a line on a second side of the N-shape;
extracting pixel coordinates ***p*₁,*p*₂,*p*₃** of the point P1, the point P2, and the point P3 in the coordinate system {I} of the ultrasound image, respectively;
calculating coordinates ***P*₂** of the point P2 in the coordinate system {P} of the calibration module according to a similar-triangle principle,
wherein ${P}_{2} = \left(1-λ\right) {P}_{B 1} + λ {P}_{AZ} ; λ = \frac{\left‖{p}_{1}-{p}_{2}\right‖}{\left‖{p}_{1}-{p}_{3}\right‖} ;$
wherein two-dimensional pixel coordinates and three-dimensional coordinates of the point P2 in the model coordinate system {P} are respectively represented by ***pᵢ*** = **[*uᵢ*,*vᵢ*]^{T}** and ***Pᵢ*,** *i* = **1,···,*K*;** wherein K denotes a quantity of N-wires;
deriving the following transformation relationship according to a rotation matrix and a translation vector of the first transformation matrix $T P F$: ${s}_{x} {u}_{i} {r}_{1} + {s}_{y} {v}_{i} {r}_{2} + t = {y}_{i} ;$
wherein ***r***₁ and ***r***₂ are first two columns of a rotation component ***R*** of the second transformation matrix $T I F$, and ***t*** is a translation vector of $T I F$, ***y**ᵢ = **R P**ᵢ +**t***; let ***a***₁ *= sₓ**r***₁,***a***₂ *= s_{y}**r***₂, $x = {\left[{a}_{1}^{T}, {a}_{2}^{T}, {t}^{T}\right]}^{T}$, and the above formula is rewritten in matrix form as: ${A}_{i} x = {y}_{i}$
where: ${A}_{i} = \left[\begin{matrix}{u}_{i} & 0 & 0 & {ν}_{i} & 0 & 0 & 1 & 0 & 0 \\ 0 & {u}_{i} & 0 & 0 & {ν}_{i} & 0 & 0 & 1 & 0 \\ 0 & 0 & {u}_{i} & 0 & 0 & {ν}_{i} & 0 & 0 & 1\end{matrix}\right] ;$
stacking all ***Aᵢ*** and ***yᵢ*** (*i* = **1, ···,*K***) row-wise to form a final linear equation ***Ax*** = ***y*,** a least-squares solution thereof being ***x*** = **(*A^{T}A*)⁻¹*A^{T}y*;**
after ***x*** is determined, ***a***₁*, **a***₂, and ***t*** are obtained accordingly; obtaining ***sₓ*** = ∥***a*₁**∥, ***s_{y}*** = ∥***a*₂**∥, ***r*₁** = ***a*₁/*sₓ***, ***r*₂ = *a*₂/*s_{y}***, and ***r*₃** = ***r*₁ × *r*₂** through calculation so as to obtain the second transformation matrix.

5. The calibration method according to claim 4, **characterized in that** after obtaining ***sₓ*** = ∥***a*₁**∥, ***s_{y}*** = ∥***a*₂**∥, ***r*₁ = *a*₁/*sₓ*, *r*₂** = ***a*₂/*s_{y}***, and ***r*₃ = *r*₁ × *r*₂** through calculation so as to calculate the second transformation matrix, the calibration method further comprises:
calculating a root-mean-square error $RMSE = \sqrt{\frac{1}{n} {\sum }_{i = 1}^{n} {\left‖{{T}_{FI}}^{I}{P}_{i}-P i F\right‖}^{2}}$;
defining outliers according to {∪***P**ᵢ*| ∥***T**_{FP} ^{P}**P**ᵢ* - *^{F}**P**ᵢ*∥ *> RMSE*}, and removing outliers satisfying the outlier definition formula from original data;
recalculating *sₓ* and *s_{y}*, and recalculating to obtain ***T**_{FP}.*

6. A calibration device for a surgical operation device, **characterized in that** the calibration device for a surgical operation device comprises:
an acquisition module configured to obtain a first transformation matrix $T P F$ between a base coordinate system {F} of the surgical operation device and a coordinate system {P} of a calibration module mounted on a base of the surgical operation device;
an ultrasound imaging module configured to perform ultrasound imaging of the calibration module using an ultrasound probe of the surgical operation device and to extract features from an ultrasound image; and
a computation module configured to map the features of the ultrasound image to corresponding actual three-dimensional features to obtain a second transformation matrix $T I F$ between a coordinate system {I} of the ultrasound image and the base coordinate system {F} of the surgical operation device, and determine, based on the second transformation matrix $T l F$, a positional deviation of the base of the surgical operation device relative to the physical space of the ultrasound probe.

7. The calibration device according to claim 6, **characterized in that** the computation module comprises:
a first setting module configured to set pixel scaling factors of the ultrasound image as Sx and Sy;
a second setting module configured to set **(*u*,*v*)** as pixel coordinates in the coordinate system {I} of the ultrasound image, and set **(*x*, *y*, *z*)** as three-dimensional coordinates corresponding to **(*u*,*v*)** in the coordinate system {P} of the calibration module:
a formula establishment module, configured to establish the following formula: $\left[\begin{matrix}x \\ y \\ z \\ 1\end{matrix}\right] = T - 1 P F T I F \left[\begin{matrix}{s}_{x} u \\ {s}_{y} u \\ 0 \\ 1\end{matrix}\right] .$

8. A non-volatile storage medium, **characterized in that** the non-volatile storage medium stores a plurality of instructions, the instructions being configured to be loaded and executed by a processor so as to cause the processor to perform the calibration method according to any one of claims 1 to 5.

9. A surgical operation device, **characterized in that** the surgical operation device is configured for use with the calibration method according to any one of claims 1 to 5, comprising:
an operation member (10) movably disposed;
an ultrasound probe (20) mounted on the operation member (10); and
a calibration module (30) detachably mounted on the operation member (10), wherein the calibration module (30) comprises a calibration wire (31) having a plurality of N-shaped structures, and the calibration wire (31) is disposed opposite the ultrasound probe (20).

10. The surgical operation device according to claim 9, **characterized in that** the calibration module (30) further comprises:
a mounting body (32) detachably mounted on the operation member (10), the mounting body (32) having a first mounting side wall and a second mounting side wall oppositely disposed, wherein the first mounting side wall is provided with a plurality of first threading holes, the second mounting side wall is provided with a plurality of second threading holes, and the calibration wire (31) is threaded back and forth between the plurality of first threading holes and the plurality of second threading holes to form a plurality of N-shaped structures.

11. The surgical operation device according to claim 10, **characterized in that** the calibration module (30) further comprises:
a first detachable arm (33) and a second detachable arm (34) spaced apart from each other, wherein the first detachable arm (33) and the second detachable arm (34) are oppositely disposed on two sides of the mounting body (32), respectively configured to be snap-fitted to two sides of the ultrasound probe (20) and detachably connected thereto, and the mounting body (32) is positioned below a detection head of the ultrasound probe (20).

12. The surgical operation device according to claim 9, **characterized in that** the ultrasound probe (20) comprises a mounting member (21) and a probe portion (22) connected to each other, the mounting member (21) being provided with a mounting channel (211), the probe portion (22) being mounted on the mounting member (21), a portion of the probe portion (22) being within the mounting channel (211), and another portion thereof extending out of the mounting channel (211); the mounting member (21) comprises a fixed end and an openable end spaced apart from each other, the openable end being rotatably disposed relative to the fixed end;
wherein contact surfaces of the fixed end and the openable end coincide with a central plane of the probe portion (22); and/or
the surgical operation device further comprises a puncture needle (40), the puncture needle (40) being positioned on the central plane of the probe portion (22).

13. The surgical operation device according to claim 9, **characterized in that** the surgical operation device further comprises a puncture needle (40); the operation member (10) comprises:
an angle control module (11), wherein a drive portion of the angle control module (11) is rotatably disposed relative to a body portion (111) of the angle control module (11), and the ultrasound probe (20) is mounted on the body portion (111) of the angle control module (11); and
a depth control module (12), wherein the drive portion of the angle control module (11) is drivingly connected to the depth control module (12), a drive end of the depth control module (12) is movably disposed along a preset direction, and the drive end of the depth control module (12) is drivingly connected to the puncture needle (40).

14. The surgical operation device according to claim 13, **characterized in that**
the angle control module (11) comprises a slider (112) and a connecting rod (113), wherein the slider (112) is movably arranged on a body portion (111) of the angle control module (11) along a preset direction; the connecting rod (113) forms a drive portion of the angle control module (11), wherein one end of the connecting rod (113) is hinged to the slider (112) and the other end thereof is hinged to the depth control module (12); and/or
the depth control module (12) comprises a lead screw guide rail module (121) and a drive block (122), wherein the lead screw guide rail module (121) is rotatably arranged, the drive block (122) is mounted on the lead screw guide rail module (121) and is threadedly engaged therewith, such that rotation of the lead screw guide rail module (121) drives the drive block (122) to move along a preset direction.

15. The surgical operation device according to claim 13, **characterized in that** the surgical operation device further comprises a micro-adjustment control module (50) mounted on the operation member (10), wherein the micro-adjustment control module (50) is configured to adjust the angle control module (11) and the depth control module (12); and/or
the operation member (10) has an initial state and an operational state for puncture; when the operation member (10) is in the initial state, the angle control module (11) and the depth control module (12) are arranged in abutment with each other.

16. The surgical operation device according to claim 9, **characterized in that** the surgical operation device further comprises a puncture needle (40) and a quick-release structure (60), wherein the puncture needle (40) is mounted on the quick-release structure (60), and the quick-release structure (60) is detachably mounted on the operation member (10).

17. The surgical operation device according to claim 16, **characterized in that** the quick-release structure (60) comprises a base (61) and a quick-release plate (62), wherein the quick-release plate (62) is movably disposed on the base (61) so as to move between a clamping position, in which the quick-release plate is disposed opposite at least a portion of the base and configured to clamp the puncture needle (40), and a release position, in which the quick-release plate is displaced away from the base;
wherein the base (61) is provided with a first snap-fit structure (63), and the quick-release plate (62) is provided with a second snap-fit structure (64) adapted to the first snap-fit structure (63); when the quick-release plate (62) is in the clamping position, the first snap-fit structure (63) is snap-engaged with the second snap-fit structure (64); the quick-release structure (60) further comprises an elastic member (65), wherein the elastic member (65) is disposed on the first snap-fit structure (63) or the second snap-fit structure (64), and is positioned between the first snap-fit structure (63) and the second snap-fit structure (64); and/or
the puncture needle (40) comprises a mounting portion (41) and a needle body (42), wherein the base (61) is provided with a first mounting groove (611) adapted to the mounting portion (41), and the quick-release plate (62) is provided with a second mounting groove (621) adapted to the mounting portion (41); when the quick-release plate (62) moves to the clamping position, the first mounting groove (611) and the second mounting groove (621) are joined together to form an enclosure around the periphery of the mounting portion (41), and the needle body (42) extends out of the quick-release structure (60).

18. The surgical operation device according to claim 16, **characterized in that** the surgical operation device further comprises:
an isolation plate (70) detachably mounted on the operation member (10), wherein the puncture needle (40) is detachably mounted on a side of the isolation plate (70) away from the operation member (10).
